# EUROPÄISCHE PATENTANMELDUNG

(11) **EP 2 277 398 A1**
(43) Veröffentlichungstag der Anmeldung: **26.01.2011**
(21) Anmeldenummer: 09166122.3
(22) Anmeldetag: 22.07.2009
(51) Int. Cl.: A24F 47/00, A61M 15/06

(54) **Rauchfreies Zigarettenersatzprodukt**

(71) Anmelder: Wedegree GmbH, 20357 Hamburg (DE)
(72) Erfinder: Rinker, Arno, 20251 Hamburg (DE); Litzenberger, Philipp, 20251 Hamburg (DE)
(74) Vertreter: Office Freylinger

(57) **Zusammenfassung**

Verfahren und Vorrichtung zum Verflüchtigen von Wirk- und/oder Aromastoffen zwecks Abgabe eines inhalierbaren Aerosols, wobei ein Fluid einen Strömungskanal in einem vorzugsweise zylindrischen Hohlkörper durchströmt und wobei das Fluid in diesem Strömungskanal mindestens eine Verdampfermembran teilweise oder vollständig um- und/oder durchströmt, wobei die mindestens eine Verdampfermembran mit einer zu verdampfenden Wirk- und oder Aromastoff enthaltenden Substanz benetzt ist und/oder benetzt wird und wobei das ggf. zusätzlich Wärmeenergie beinhaltende Fluid beim Durchströmen und/oder Umströmen der Verdampfermembran diese Substanz oder Substanzen verdampft und/oder zerstäubt und dem Fluidstrom zuführt.

## Beschreibung

### Technisches Gebiet

Die vorliegende Erfindung betrifft allgemein ein Verfahren und eine Vorrichtung zur rauchfreien Inhalation von Nikotin und/oder Zusatzstoffen.

### Allgemeine Einleitung

Beim Rauchen einer herkömmlichen Zigarette wird Tabak verbrannt und der bei dieser Verbrennung entstehende Rauch wird inhaliert (Hauptstromrauch) oder an die Umgebung abgegeben (Nebenstromrauch).

Der Hauptstromrauch ist maßgeblich für die Gesundheitsschädigung des Konsumenten verantwortlich, liefert ihm aber den gewünschten Genuss. Der Nebenstromrauch ist maßgeblich für die Gesundheitsschädigung der Passivraucher verantwortlich und ist weder von den Passivrauchern noch vom Konsumenten gewünscht.

Im Rauch einer Zigarette wurden über 4800 verschiedene Substanzen nachgewiesen von denen ungefähr 70 als nachweislich krebserregend gelten.

Verfahren und darauf basierende Vorrichtungen zur rauchfreien Inhalation von Nikotin und/oder Zusatzstoffen haben zum Ziel dem Konsumenten ein Substitut zur herkömmlichen Zigarette zu bieten, welches eine wesentlich geringere Gesundheitsschädigung beim Konsumenten und Dritten, vorzugsweise gar keine Gesundheitsschädigung beim Konsumenten und Dritten verursacht, dabei jedoch das Konsumerlebnis einer herkömmlichen Zigarette beibehält.

### Stand der Technik

Die bekannten Verfahren und Vorrichtungen beschreiben eine Vielzahl von Möglichkeiten eine Substanz vorzugsweise eine Mixtur aus Nikotin und Zusatzstoffe zu verdampfen um ein inhalierbares Aerosol zu erzeugen.

Hierbei wird in Abhängigkeit vom Dampfdruck der Substanz diese entweder passiv nur durch einen Fluidstrom oder aktiv unter zusätzlicher Zuführung von z. B. von Wärmeenergie verdampft.

AT414084BB beschreibt z. B. eine Kartonröhre aus Pappe oder biologisch abbaubarem Kunststoff, welche in einer weiteren Ausgestaltung durch eine Alufolie oder Kunststofffolie von innen luftundurchlässig beschichtet ist. Ein innerhalb dieser Röhre angeordneter Trägerkörper in Form von z. B. treppenförmigen Lamellen bringt einen die Vorrichtung durchströmenden Fluidstrom mit einer zu Substanz in Kontakt, die so dem Fluidstrom zugeführt wird.

DE202005006312U1 beschreibt ein Plastik oder Kunststoffrohr mit Sollknickstelle und einem innenliegenden porösen oder hohlen Tränkkörper der vorzugsweise zylinderförmig und z. B. aus Baumwolle ist. Dieser Tränkkörper soll eine Substanz an einen Fluidstrom abgeben.

DE202006001663U1 beschreibt ein zweiteiliges Zigarettenröhrchen aus Kunststoff, Papier oder Metall in welchem sich ein mit einer Substanz getränktes Kissen zwischen zwei Membranventilen befindet, wobei sich die Membranventile durch einen Fluidstrom öffnen und das Kissen dabei die Substanz an den Fluidstrom abgibt.

DE2939965C2 beschreibt ebenfalls ein Röhrchen in dem ein mit einer Substanz beaufschlagtes Kissen so in einem Fluidstrom angeordnet wird, dass durch die Oberfläche des Kissens ein Venturikanal gebildet wird, wobei der so durch einen Fluidstrom erzeugte Unterdruck die Verdampfung unterstützt.

DE69130682T3 beschreibt eine Patrone für einen Nikotininhalator bestehend aus einer Copolymer-Gehäusehülse und einem darin befindlichen Nikotinbehälter, welcher vorzugsweise als poröser Polymerstopfen ausgeführt ist, welcher beim Durchströmen Nikotin an einen Fluidstrom abgibt. Diese Erfindung wird aktuell als "Nicorette Inhaler" vertrieben.

WO2005/099494A1 beschreibt eine Vorrichtung zur rauchfreien Inhalation von Nikotin, wobei eine nikotinhaltige Substanz in einem Vlieskissen gespeichert und zum Verdampfen an einen Ultraschallzerstäuber abgegeben wird. Dieses Produkt wird als sogenannte "E-Cigarette" von verschiedenen Anbietern vertrieben.

Allen bekannten Verfahren und Vorrichtungen zur rauchfreien Inhalation von Nikotin und/oder Zusatzstoffen ist gemein, dass die Speicherung der jeweiligen Substanz in einem dreidimensional ausgeprägten Medium, vorzugsweise einem ein- oder mehrteiligen Kissen oder zylindrischen saugfähigen und/oder porösen Körper erfolgt, wobei diese dreidimensional ausgeprägten Medien entweder durch die Durch- und/oder Umströmung eines Fluides und ggf. direkte und/oder indirekt Zufuhr von Wärmeenergie neben ihrer Speicherfunktion auch als Verdampfer agieren oder die enthaltene Substanz an eine zusätzliche Verdampfervorrichtung abführen.

Nachteilig bei allen bekannten Verfahren und Vorrichtungen zur rauchfreien Inhalation von Nikotin und/oder Zusatzstoffen die die Substanz einer externen Verdampfervorrichtung zuführen, ist zum einen die damit verbundene unerwünschte Verunreinigung der Verdampfervorrichtung, wie sie von sogenannten E-Cigarettes (WO2005/099494A1) bekannt ist und zum anderen die Dosierbarkeit der Substanz, wodurch die Verdampfervorrichtung, unter anderem abhängig von ihrer jeweiligen räumlichen Lage, häufig entweder über- oder unterversorgt wird.

Nachteilig bei allen bekannten Verfahren und Vorrichtungen, die das Speichermedium auch als Verdampfer benutzen, ist ein unzureichender Wirkungsgrad der Verdampferfunktion wodurch die Anreicherung des Fluidstroms mit einer gewünschten Substanz nicht in gewünschtem Umfang dargestellt werden kann oder einer unnötig hohen direkt und/oder indirekt zugeführten Wärmeenergie bedarf.

Ein weiterer Nachteil bei allen bekannten Verfahren und Vorrichtungen, die das Speichermedium auch als Verdampfer benutzen, ist häufig die mit fortlaufender Konsumdauer abnehmende Intensität der Verdampfung, da sich mit fortschreitender Verdampfung zwangsläufig die Sättigung des Speichermediums und somit die Sättigung des Verdampfers verringert.

Die genannten Nachteile führen dazu, dass dem Konsumenten durch alle bekannten Verfahren und Vorrichtungen zur rauchfreien Inhalation von Nikotin und/oder Zusatzstoffen entweder ein zu schwaches und/oder zu ungleichmäßiges Inhalationserlebnis oder ein zwar ausreichend starkes und/oder gleichmäßiges Inhalationserlebnis unter zu hohem technischen und/oder energetischen Aufwand geboten wird.

### Aufgabe der Erfindung

Eine Aufgabe der vorliegenden Erfindung ist es demnach ein Verfahren und eine darauf basierende Vorrichtung zur rauchfreien Inhalation von Nikotin und/oder Zusatzstoffen bereit zu stellen, die eine Verdampfung von Nikotin und/oder Zusatzstoffen mit deutlich erhöhtem Wirkungsgrad gegenüber dem Stand der Technik ermöglicht.

### Allgemeine Beschreibung der Erfindung

Diese Aufgabe wird erfindungsgemäß gelöst durch die Bereitstellung eines Verfahrens zum Verflüchtigen von Wirk- und/oder Aromastoffen zwecks Abgabe eines inhalierbaren Aerosols, wobei ein Fluid einen Strömungskanal 11 in einem vorzugsweise zylindrischen Hohlkörper 1 durchströmt und wobei das Fluid in diesem Strömungskanal 11 mindestens eine Verdampfermembran 2 teilweise oder vollständig um- und/oder durchströmt, wobei die mindestens eine Verdampfermembran 2 mit einer zu verdampfenden Wirk- und oder Aromastoff enthaltenden Substanz benetzt ist und/oder benetzt wird und wobei das ggf. zusätzlich Wärmeenergie beinhaltende Fluid beim Durchströmen und/oder Umströmen der Verdampfermembran 2 diese Substanz oder Substanzen verdampft und/oder zerstäubt und dem Fluidstrom zuführt.

Die in diesem Dokument verwendeten Referenzzeichen dienen nur Illustrationszwecken und sind nicht als Limitierung auf die in den Figuren gezeigten Ausgestaltungen zu verstehen.

Ein solches Verfahren, vorzugsweise zum Inhalieren einer Nikotin-Aroma-Mixtur, wird ermöglicht durch die Anordnung mindestens einer zu verdampfenden vorzugsweise Nikotin und/oder Aromen enthaltenden Substanz auf mindestens einer permeablen oder semipermeablen, d. h. durchlässigen oder teildurchlässigen Verdampfermembran 2 in einem Fluidstrom, wobei durch dieses Verfahren ein sehr großes Verhältnis von Fluid-Substanz-Kontaktfläche zu Verdampfermasse (Masse der Membran) bei gleichzeitig geringem Strömungswiderstand zu erzielen ist. Dabei ist der Vorteil eines solchen Verfahrens im Gegensatz zu Verfahren bei denen eine zu verdampfende vorzugsweise Nikotin und/oder Aromen enthaltenden Substanz auf ein dreidimensional ausgeprägtes Medium, z. B. ein Kissen oder einen zylindrischen saugfähigen und/oder porösen Körper aufgebracht ist, dass der Wirkungsgrad, d. h. das Verhältnis von Nikotininput zu Nikotinoutput prinzipbedingt höher ist.

In einer weiteren Ausgestaltung umfasst das Verfahren die Verwendung mindestens einer Filtermembran 4, wobei die Filtermembran 4 semipermeabel bzw. selektiv permeabel, d. h. nicht für alle Substanzen durchlässig ist, um dem Fluidstrom unerwünschte Substanzen gezielt zu entziehen ohne den Strömungswiderstand unnötig zu erhöhen. Der Vorteil einer solchen Ausgestaltung liegt in der Möglichkeit z. B. durch eine Anordnung nach einer Verdampfermembran 2 eventuell zu große gelöste Tröpfchen der zu verdampfenden Substanz aus dem Fluidstrom zu entfernen oder bei Verwendung des Verfahrens in Kombination mit einem Fluid erwärmenden Verfahren von diesem erzeugte unerwünschte Substanzen zu entfernen. Auch die Anordnung vor einer Verdampfermembran 2 oder die kombinierte Anordnung vor und nach einer Verdampfermembran 2 sind ausdrücklich vorgesehen. Dies ist z. B. dann der Fall wenn mehrere Verdampfermembranen 2 im Strömungskanal 11 angeordnet sind, wobei jede für die Verdampfung unterschiedlicher Wirk- und/oder Aromastoffe enthaltenden Substanzen vorgesehen ist, die Filterfunktion sich aber auf eine oder einige dieser Substanzen beschränken soll.

In einer weiteren Ausgestaltung ist mindestens eine Filtermembran 5 mit einem Katalysatormaterial beaufschlagt, welches eventuell vorhandene schädliche Bestandteile des Fluidstroms in gesundheitlich unbedenkliche Bestandteile zerlegt.

Die Vorteile eines solchen Verfahrens liegen zum einen in der Möglichkeit bei hohem Wirkungsgrad und geringem Strömungswiderstand einen Fluidstrom gezielt mit erwünschten Substanzen anzureichern und zum anderen aus diesem unerwünschte Substanzen zu entfernen.

Die obengenannte Membran oder Membranen, d.h. sowohl Verdampfermembran(en) 2 und/oder Filtermembran(en) 4, 5, können auch selbst aus ein- oder mehrschichtigen Membran(en), permeablen oder semipermeablen Membranen bestehen oder Kombinationen davon enthalten. Die Membranen bestehen aus an sich bekannten Materialien, z. B. aus Vliesstoffen, Geweben, Gestricken und/oder Gewirken. Bevorzugt als Material für Verdampfermembranen 2 benutzt man Vliesstoffe aus Zelluloseregeneratfasern und für Filtermembranen 4, 5 eignen sich besonders Vliesstoffe aus Abacäfasern.

Die erfindungsgemäß einsetzbaren Membranen 2, 4, 5 können orthogonal, winklig und/oder parallel zum Fluidstrom angeordnet sein, wobei auch dreidimensionale Topologien oder Gebilde (z. B. Hohlkegel, Falten) vorgesehen sein können. Bei der Verdampfermembran 2 ist es wichtig, dass sie einen größtmöglichen Kontakt zum Fluid erlaubt, sowohl durch durchströmen wie durch umströmen. Prinzipbedingt ist es für eine Filtermembran 4, 5 natürlich wichtig, dass der Fluidstrom im Wesentlichen durch sie hindurch strömt.

In einer weiteren bevorzugten Ausgestaltung werden die Verdampfermembran(en) 2 über Kapillare 3 aus mindestens einem, vorzugsweise mit einem Speichermedium 64, z. B. einem Schaum oder Vlies gefüllten Hohlraum 6 über Versorgungsöffnungen 61 in der Hohlraumwandung mit zu verdampfender Flüssigkeit versorgt. Dadurch ist es möglich die Verdampferfunktion von der Speicherfunktion zu entkoppeln.

Der Vorteil dieser Ausgestaltung besteht in der Darstellbarkeit einer stets optimalen Benetzung der Verdampfermembran(en) 2 bei gleichzeitig optimal auf die Wünsche des Konsumenten abstimmbarer Menge an zu verdampfender Substanz pro Konsumakt. Es wird dadurch gleichzeitig eine möglichst gleichmäßige Verdampfung über mehrere Züge gewährleistet.

Ein weiterer Vorteil dieser Variante ist es, dass Wirk- und/oder Aromastoffe, z. B. Nikotin und/oder andere Zusatzstoffe, bis zum Konsum vor Atmosphäreneinflüssen geschützt sind, und somit eine möglichst lange Haltbarkeit erzielt wird. Außerdem kann dadurch auch gewährleistet werden, dass Stoffe mit relativ verschiedenen Flüchtigkeiten beim Konsum in der vorgesehenen Kombination zur Verfügung stehen.

In einer weiteren bevorzugten Ausgestaltung umfasst (umfassen) der Hohlraum (die Hohlräume) 6 mindestens eine zusätzliche Druckausgleichsöffnung 62 in der Hohlraumwandung wodurch der Hohlraum 6 mit dem Strömungskanal 11 und/oder der Atmosphäre in Verbindung steht, wodurch ein Druckausgleich beim Abführen der im Hohlraum 6 gespeicherten zu verdampfenden Substanz gewährleistet ist und diese so einfacher über die Kapillare 3 auf die Verdampfermembran(en) 2 transportiert werden kann. Zusätzlich können der Fluidstrom und durch diesen verursachte Druckdifferenzen auf diese Weise den Transport der zu verdampfenden Substanz aus dem Hohlraum (den Hohlräumen) 6 auf die Verdampfermembran(en) 2 unterstützen. Die zu verdampfende Substanz kann den Hohlraum, bzw. die Hohlräume 6 teilweise, vorzugsweise aber vollständig ausfüllen.

Die Druckausgleichsöffnung(en) 62 ist (sind) vorzugsweise so gestaltet, dass die zu verdampfende Substanz z. B. aufgrund ihrer Oberflächenspannung, nicht in der Lage ist die Druckausgleichsöffnung(en) 62 zu passieren, wodurch ein unerwünschtes Austreten der zu verdampfenden Substanz vermieden wird.

In einer weiteren Ausgestaltung sind die Versorgungsöffnung(en) 61 durch einen oder mehrere Versorgungsöffnungsverschlüsse 611 und/oder Druckausgleichsöffnung(en) 62 durch einen oder mehrere Druckausgleichsöffnungsverschlüsse 622 verschlossen und werden erst vor dem Konsum, z. B. durch aufreißen, perforieren oder verschieben, geöffnet. Auf diese Weise kann die zu verdampfende Substanz bis zum Konsum vor Atmosphäreneinflüssen geschützt und an einer ungewollten vorzeitigen Verflüchtigung verhindert werden.

Eine weitere Möglichkeit die zu verdampfende Substanz bis zum Konsum vor Atmosphäreneinflüssen zu schützen und an einer ungewollten vorzeitigen Verflüchtigung zu hindern ist die Unterteilung des Hohlraums (der Hohlräume) 6 in mindestens eine Substanzversorgungskammer 631 und eine Substanzspeicherkammer 632, wobei diese durch eine entfernbare Trennwand 63 voneinander getrennt sind und die Substanzspeicherkammer 632 keine weiteren Öffnungen außer die durch die entfernbare Trennwand 63 verschlossene Öffnung zur Substanzversorgungskammer 631 aufweist.

Das beschriebene Verfahren wird bevorzugt ausgeführt mit einer erfindungsgemäßen Vorrichtung zur Verdampfung von Wirk- und/oder Aromastoffen mit möglichst hohem Wirkungsgrad, d. h. mit möglichst hohem Verhältnis von Wirk- und/oder Aromastoff-Input zu Wirk- und/oder Aromastoff-Output, umfassend einen vorzugsweise zylindrischen Hohlkörper 1, welcher einen Strömungskanal 11 bildet oder beinhaltet, in den Abmessungen einer kleinen Zigarre oder Zigarette, vorzugsweise in Form und Abmessungen einem Zigarettenfilter inklusive Banderole ähnlich, umfassend mindestens eine semipermeable oder permeable Verdampfermembran 2, die im Strömungskanal 11 angeordnet ist, zum Verdampfen einer Wirk- und/oder Aromastoff enthaltenden Substanz, wobei die Substanz auf die Verdampfermembran 2 bereits aufgetragen ist (Verdampfermembran 2 benetzt) und/oder aus einem optional vorhandenen die Substanz enthaltenden Hohlraum 6 (s.u.) auf die Verdampfermembran 2 auftragbar ist (Verdampfermembran 2 benetzbar).

Die Vorrichtung bildet demnach bevorzugt ein Wirk- und/oder Aromastoff-Depot enthaltendes Mundstück, welches vorteilhafterweise eine zylindrische Hülse 1 umfasst, mit einem oder mehreren Fluideingängen 111 und einem oder mehreren Fluidausgängen 112 wobei durch den Sog eines Benutzers an dem Luftausgang (den Luftausgängen) 112 ein Fluidstrom im Strömungskanal 11 entsteht. Diese Hülse 1 ist im Prinzip Haltefläche für den Konsumenten und Kontaktfläche mit dem Mund des Konsumenten.

In einer weiteren Ausführungsform beinhaltet die Vorrichtung vorzugsweise mindestens eine semipermeable Filtermembran 4 zum Entfernen unerwünschter Bestandteile aus dem Fluidstrom. Optional kann alternativ oder zusätzlich mindestens eine semipermeable oder permeable Filtermembran 5 mit einem aufgebrachten Katalysator vorgesehen sein zum Aufspalten von unerwünschten Bestandteilen des Fluidstroms in gesundheitlich unbedenkliche Bestandteile,

In einer weiteren bevorzugten Ausgestaltung sind Kapillare 3 zum Nachführen einer zu verdampfenden Substanz auf die Verdampfermembran(en) 2 vorgesehen.

Vorzugsweise ist mindestens ein Hohlraum 6 zum Speichern einer oder mehrerer zu verdampfenden Substanz(en) innerhalb der Vorrichtung vorgesehen, welcher über mindestens eine Versorgungsöffnung 61 mit den Kapillaren 3 und/oder Verdampfermembranen 2 in Verbindung steht und vorzugsweise ein Speichermedium 64, z. B. einen Schaum oder ein Vlies enthält, um ein unerwünschtes (vorzeitiges) Entweichen der zu verdampfenden Substanz(en) zu erschweren, wobei die Öffnung(en) vorzugsweise vor Gebrauch durch z. B. mindestens einen entfernbaren Versorgungsöffnungsverschluss 611 verschlossen ist (sind), um die vor Gebrauch im Hohlraum (in den Hohlräumen) 6 befindliche zu verdampfende(n) Substanz(en) vor Atmosphäreneinflüssen und frühzeitiger Verflüchtigung zu schützen.

Wie oben bereits beschrieben können Hohlkörper 1 und falls vorhanden die Hohlräume 6 aus einer oder mehreren ein- und/oder mehrlagigen Folien gebildet sein.

Weiterhin ist vorzugsweise mindestens eine Druckausgleichsöffnung 62 vorgesehen, die den Hohlraum (die Hohlräume) 6 mit dem Strömungskanal 11 und/oder der Atmosphäre verbindet, um das Nachführen der zu verdampfenden Substanz(en) über die Kapillare 3 auf die Verdampfermembran(en) 2 zu unterstützen, wobei die Druckausgleichsöffnung(en) 62 vorzugsweise vor Gebrauch durch mindestens einen entfernbaren Druckausgleichsöffnungsverschluss 621 verschlossen ist (sind), um die vor Gebrauch im Hohlraum (in den Hohlräumen) 6 befindliche zu verdampfende(n) Substanz(en) vor Atmosphäreneinflüssen und frühzeitiger Verflüchtigung zu schützen.

In einer weiteren Ausführungsform ist vorzugsweise weiterhin mindestens eine entfernbare Trennwand 63 ausgebildet, die den Hohlraum 6 in mindestens eine Substanzversorgungskammer 631 und eine Substanzspeicherkammer 632 unterteilt, wobei die Substanzspeicherkammer(n) 632 keine weiteren Öffnungen als die durch die Trennwand 63 verschlossenen umfasst und die Trennwand 63 vor Gebrauch entfernt und/oder zerstört wird und die zu verdampfende Substanz so aus der (den) Substanzspeicherkammer(n) 632 in die Substanzversorgungskammer(n) 631 innerhalb des Hohlraums 6 fließt.

Bevorzugt ist ein Flansch 7 zum Verbinden der erfindungsgemäßen Vorrichtung mit einer weiteren Vorrichtung, wie z. B. ein Heizstab wie in WO2008/113420 beschrieben vorgesehen, wobei der Lufteingang (die Lufteingänge) des Strömungskanals 11 mit den Luftausgängen dieser weiteren Vorrichtung in Verbindung stehen und diese weitere Vorrichtung vorzugsweise in der Lage ist einem Fluidstrom Energie vorzugsweise in Form von Wärmeenergie zuzuführen, um ggf. den Verdampfungsprozess einer schwer zu verflüchtigenden Substanz zu unterstützen.

Die Vorteile eines solchen Verfahrens und einer darauf basierenden Vorrichtung sind vielfältig. Es erfolgt erstens keine Rauchbelästigung durch Verbrennung komplexer Stoffe, wie Tabak, und zweitens entsteht kein so genannter Nebenstromrauch, wodurch Dritte nicht durch Passivrauchen geschädigt werden. Drittens, dadurch dass im Gegensatz zu einer herkömmlichen Zigarette die Wirk- und/oder Aromastoffe und deren Anzahl gezielt ausgewählt werden können, ist deren Inhalation gesundheitlich bei weitem unbedenklicher als bei einer normalen Zigarette und so kann eine krebserregende (karzinogene) Wirkung gezielt ausgeschlossen werden. Anders ausgedrückt, da keine Verbrennung im herkömmlichen Sinne erfolgt, wird eine kontrollierte Aerosolzusammensetzung ermöglicht, ohne Dritte zu belästigen.

Dieser für die Akzeptanz vom Benutzer entscheidende Vorteil wird durch die Verflüchtigung der Wirk- und/oder Aromastoffe (Bildung eines inhalierbaren Aerosols) ausschließlich durch einen Fluidstrom, ggf. einen warmen Fluidstrom erreicht, d.h. ohne die zu verdampfende(n) Substanz(en) durch direkten Kontakt mit einer Energiequelle zu erhitzen oder gar zu verbrennen. Dabei ist ein inhalierbares Aerosol im Sinne dieser Erfindung im Prinzip ein Gemisch aus festen und/oder flüssigen Schwebeteilchen und Luft. Bevorzugt handelt es sich beim inhalierbaren Aerosol um einen Nebel, d.h. ein Gemisch bestehend hauptsächlich aus flüssigen Schwebeteilchen und Luft, vorzugsweise weitestgehend frei von Feinstaub.

Im Gegensatz zu bekannten Verfahren und darauf basierenden Vorrichtungen, bietet das vorliegende Verfahren und die darauf basierende Vorrichtung die Möglichkeit den von der herkömmlichen Zigarette bekannten und von Konsumenten erwarteten Nikotin-Aroma-Transfer nicht nur teilweise sondern vollständig zu liefern und sogar zu übertreffen. Ein ausreichender Nikotin-Aroma-Transfer, d. h. die Menge und Intensität an inhalierbarem Nikotin und Aroma pro Zug, ist der Schlüsselfaktor für eine Akzeptanz durch den Konsumenten.

### Kurze Beschreibung der Figuren

Im Folgenden werden nun Vorteile und Ausgestaltungen der Erfindung anhand der beiliegenden Figuren beschrieben.

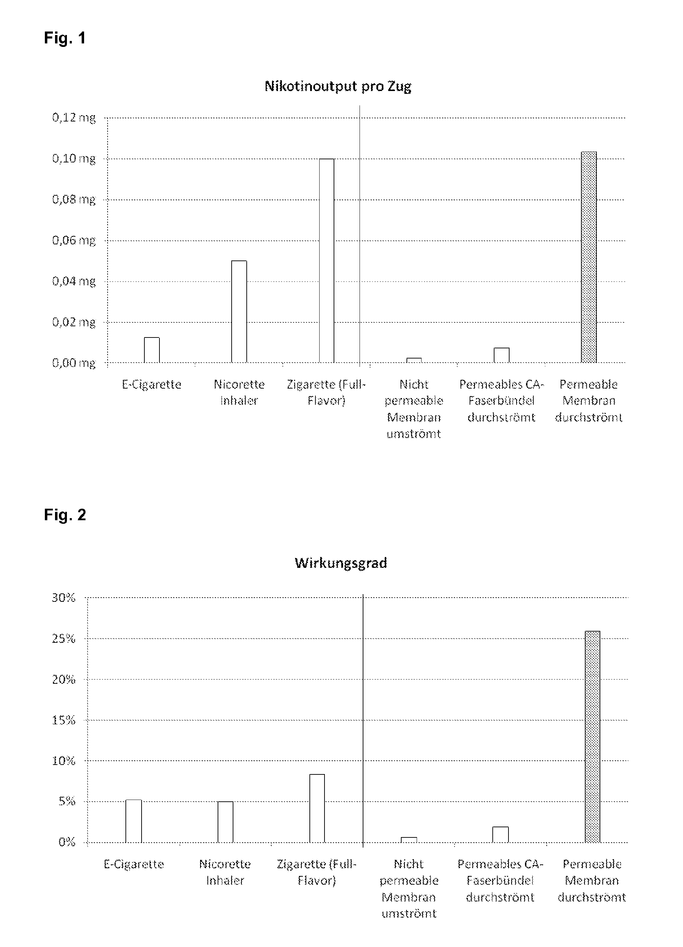

Fig. 1 zeigt zum Vergleich den Nikotinoutput pro Zug (Zugvolumen 35 ml, Zugdauer 2 s) der E-Cigarette, des Nicorette Inhalers und der herkömmlichen Full-Flavor-Zigarette auf der linken Seite (erhältliche Produkte) und jeweils einer mit der gleichen Menge an zu verdampfender Substanz getränkte umströmte impermeablen (undurchlässigen) Membran, eines durchströmten Zelluloseacetat-Faserbündels (Zigarettenfilter) und einer erfindungsgemäßen durchströmten permeablen (durchlässigen) Membran 2 aus Zelluloseregeneratfasern auf der rechten Seite (Versuche).

Fig. 2 zeigt zum Vergleich den Wirkungsgrad, d. h. das Verhältnis von im System enthaltenem zu freigesetztem Nikotin der E-Cigarette, des Nicorette Inhalers und der herkömmlichen Full-Flavor-Zigarette auf der linken Seite (erhältliche Produkte) und jeweils einer mit der gleichen Menge an zu verdampfender Substanz getränkte umströmte impermeablen (undurchlässigen) Membran, eines durchströmten Zelluloseacetat-Faserbündels (Zigarettenfilter) und einer erfindungsgemäßen durchströmten permeablen (durchlässigen) Membran 2 aus Zelluloseregeneratfasern auf der rechten Seite (Versuche).

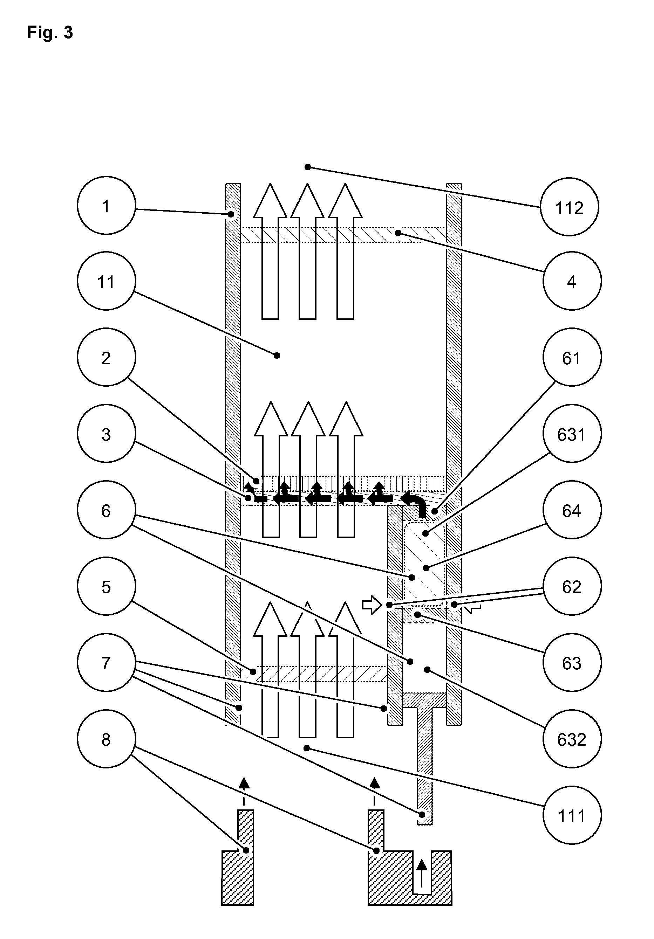

Fig. 3 zeigt eine schematische Darstellung einer bevorzugten Ausgestaltung des Verfahrens, wobei alle möglichen Bestandteile mindestens einmal aufgeführt sind.

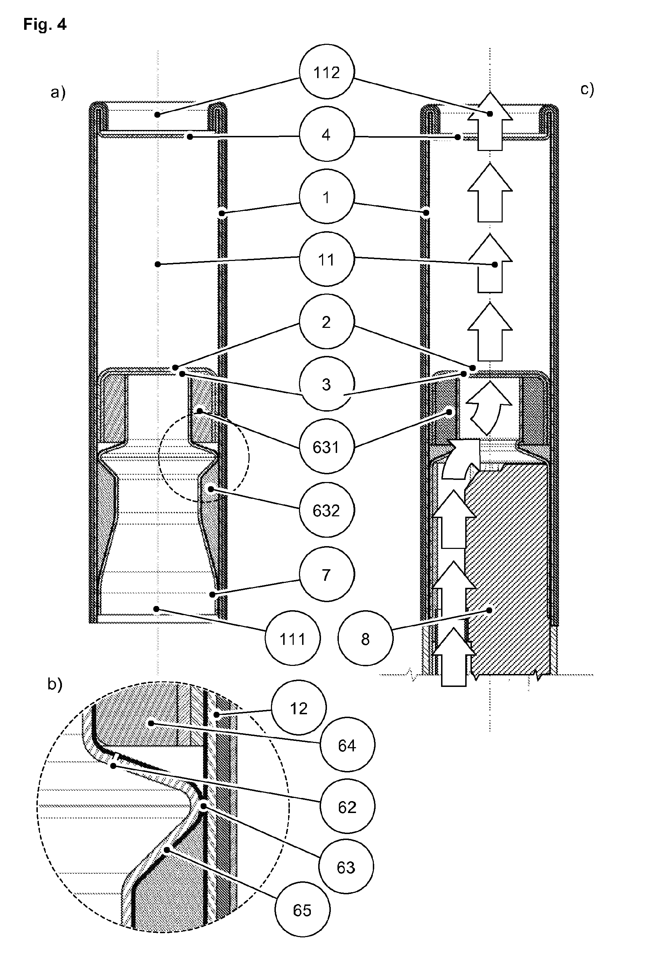

Fig. 4 zeigt Schnittzeichnungen einer vorzugsweisen Ausgestaltung einer erfindungsgemäßen Vorrichtung, wobei der Hohlkörper 1 einen Hohlraum 6 mit Substanzspeicherkammer 632 und Substanzversorgungskammer 631 aufweist und Hohlkörper 1 und Hohlraum 6 durch zwei miteinander verklebte mehrlagige Folien gebildet sind.
a) Erfindungsgemäße Vorrichtung vor dem Gebrauch. Eine zu verdampfende Substanz befindet sich in einer Substanzspeicherkammer 632, die durch einen Substanzspeicherkammerverschluss 63 in Form einer Klebenaht (Sollbruchstelle) von einer mit einem Speichermedium 64 gefüllten Substanzversorgungskammer 631 getrennt ist.
b) Detailansicht des Substanzspeicherkammerverschlusses 63 im geschlossenen Zustand und der umliegenden Bauteile mit sichtbarem Lagenaufbau der mehrlagigen Folie.
c) Erfindungsgemäße Vorrichtung fertig zum Gebrauch. Eine zusätzliche Vorrichtung zum Erwärmen eines Fluidstroms ist so in den Flansch 7 der erfindungsgemäßen Vorrichtung eingeschoben, dass die Substanzspeicherkammer 632 vollständig zusammengedrückt ist, wodurch der Substanzspeicherkammerverschluss 63 in Form einer Klebenaht aufgerissen ist und die gesamte zu verdampfende Substanz in die Substanzversorgungskammer 631, Kapillare 3 und Verdampfermembran 2 übergegangen ist.

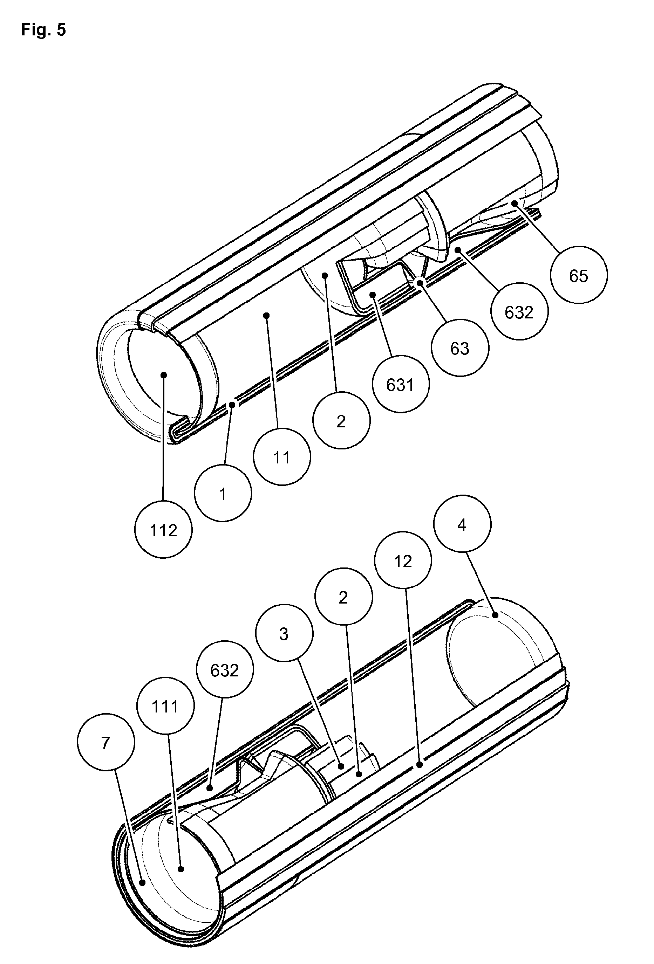

Fig. 5 zeigt ISO-Schnittansichten der Ausgestaltung einer erfindungsgemäßen Vorrichtung aus Fig. 4
a) Die Austrittsöffnung mit Filtermembran 4 liegt zum Betrachter.
b) Die Eintrittsöffnung mit Flansch 7 liegt zum Betrachter.

### Beschreibung einer bevorzugten Ausgestaltung der Erfindung

In einer bevorzugten Ausgestaltung der Erfindung dient eine erfindungsgemäße Vorrichtung als Mundstück z.B. in einer in WO2008/113420A1 beschriebenen Vorrichtung, d. h. eine erfindungsgemäße Vorrichtung wird mit einer Vorrichtung zur Abgabe von Wärmeenergie 8 an einen Fluidstrom über einen Flansch 7 verbunden.

In dieser Ausgestaltung umfasst die erfindungsgemäße Vorrichtung,
➢ eine Hülse 1 gebildet aus einer mehrlagigen Folie, vorzugsweise bestehend aus z. B. PVC-Folie, PE-Folie, Barex-Folie und/oder Aluminiumfolie, und Karton und Deckpapier, mit einem Fluideingang 111 und einem Fluidausgang 112,
➢ eine permeable Verdampfermembran 2, die im Strömungskanal 11 angeordnet ist, zum Verdampfen einer Wirk- und/oder Aromastoff enthaltenden Substanz,
➢ Kapillare 3 zum Nachführen einer zu verdampfenden Substanz auf die Verdampfermembran 2,
➢ einer der Verdampfermembran 2 nachgeschalteten semipermeablen Filtermembran 4 zum Entfernen unerwünschter Bestandteile aus dem Fluidstrom,
➢ einen durch z. B. Verkleben einer weiteren mehrlagigen Folie mit der Hülse 1 entstehenden Hohlraum 6 zum Speichern einer zu verdampfenden Substanz welcher über eine Versorgungsöffnung 61 mit den Kapillaren 3 und der Verdampfermembran 2 in Verbindung steht und ein Speichermedium 64 in Form eines Schaums enthält, um ein unerwünschtes Entweichen der zu verdampfenden Substanz zu erschweren,
➢ mehrere Druckausgleichsöffnungen 62, die den Hohlraum 6 mit dem Strömungskanal 11 verbinden, um das Nachführen der zu verdampfenden Substanz(en) über die Kapillare 3 auf die Verdampfermembran 2 zu unterstützen, wobei die Druckausgleichsöffnungen 62 durch eine Mikroperforation der Hohlraumfolie 65 gebildet werden,
➢ eine Substanzspeicherkammer 632 innerhalb des Hohlraumes 6 die durch einen Substanzspeicherkammerverschluss 63 in Form einer lösbaren Klebenaht zwischen der mehrlagigen Hülsenfolie 12 und der mehrlagigen Hohlraumfolie 65 von der Substanzversorgungskammer 631, welche die Versorgungsöffnung 61, das Speichermedium 64 und die Druckausgleichsöffnungen 62 beinhaltet getrennt ist und vor dem Gebrauch die zu verdampfende Substanz vor Atmosphäreneinflüssen und frühzeitiger Verflüchtigung schützt.
➢ einen Flansch 7 zum Verbinden der erfindungsgemäßen Vorrichtung mit einer weiteren Vorrichtung 8 z. B. einem in WO2008/113420A1 beschriebenen sogenannten Heizstab, wobei der Fluideingang 111 des Strömungskanals 11 mit dem Fluidausgang dieser weiteren Vorrichtung 8 in lösbarer Verbindung steht, wobei dieser Flansch 7 so gestaltet ist, dass die Substanzspeicherkammer 632 beim Zusammenführen der Vorrichtungen vollständig zusammengedrückt wird, wobei der Substanzspeicherkammerverschluss 63 in Form einer lösbaren Klebenaht aufreißt und die gesamte zu verdampfende Substanz in die Substanzversorgungskammer 631, Kapillare 3 und Verdampfermembran 2 überführt wird.

**Tabelle 1: Referenzliste**

| **Nr.** | **Allgemeine Bezeichnung** | **Spezifische Bezeichnung** |
|---|---|---|
| 1 | Hohlkörper | Hülse |
| 11 | Strömungskanal | |
| 111 | Fluideingang | |
| 112 | Fluidausgang | |
| 12 | Hülsenfolie | |
| 2 | Verdampfermembran | |
| 3 | Kapillare | Verdampferkapillare |
| 4 | Filtermembran | Selektive Filtermembran |
| 5 | Filtermembran | Katalytische Filtermembran |
| 6 | Hohlraum | Substanzvorrat |
| 61 | Versorgungsöffnung | |
| 611 | Verschluss | Versorgungsöffnungsverschluss |
| 62 | Druckausgleichsöffnung | Öffnung zum Druckausgleich |
| 621 | Verschluss | Druckausgleichsöffnungsverschluss |
| 63 | Trennwand | Substanzspeicherkammerverschluss |
| 631 | Substanzversorgungskammer | |
| 632 | Substanzspeicherkammer | |
| 64 | Speichermedium | |
| 65 | Hohlraumfolie | |
| 7 | Flansch | |
| 8 | Weitere Vorrichtung | Zusatzvorrichtung |

## Patentansprüche

1. Verfahren zum Verflüchtigen von Wirk- und/oder Aromastoffen zwecks Abgabe eines inhalierbaren Aerosols, wobei ein Fluid einen Strömungskanal (11) in einem vorzugsweise zylindrischen Hohlkörper (1) durchströmt und wobei das Fluid in diesem Strömungskanal (11) mindestens eine Verdampfermembran (2) teilweise oder vollständig um- und/oder durchströmt, wobei die mindestens eine Verdampfermembran (2) mit einer zu verdampfenden Wirk- und oder Aromastoff enthaltenden Substanz benetzt ist und/oder benetzt wird und wobei das ggf. zusätzlich Wärmeenergie beinhaltende Fluid beim Durchströmen und/oder Umströmen der Verdampfermembran (2) diese Substanz oder Substanzen verdampft und/oder zerstäubt und dem Fluidstrom zuführt.

2. Verfahren nach Anspruch 1, wobei im Strömungskanal (11) mindestens eine Filtermembran (4, 5) angeordnet ist, die unerwünschte Bestandteile aus dem Fluidstrom herausfiltert und/oder mittels eines Katalysators in unbedenkliche Produkte aufspaltet.

3. Verfahren nach Anspruch 1 oder 2, wobei die Membran(en) (2, 4, 5) aus ein- oder mehrschichtigen Membran(en), permeablen oder semipermeablen Membranen oder Kombination davon ausgewählt ist(sind) und orthogonal, winklig und/oder parallel zum Fluidstrom angeordnet ist(sind).

4. Verfahren nach einem der Ansprüche 1, 2 oder 3, wobei das Benetzen der Verdampfermembran(en) (2) mit einer zu verdampfenden Substanz mittels Kapillareffekt erfolgt, wobei die Kapillare (3) mit der(den) Verdampfermembran(en) (2) in Kontakt stehen und/oder in dieser(diesen) enthalten sind und wobei die Verdampfermembran(en) (2) und/oder Kapillare (3) weiterhin Kontakt zu einer zu verdampfenden Substanz erhalten.

5. Verfahren nach Anspruch 4, wobei sich die mit der oder den Verdampfermembranen (2) und/oder Kapillaren (3) in Kontakt stehende zu verdampfende Substanz in einem Hohlraum (6) oder mehreren Hohlräumen (6) befindet, wobei der oder die Hohlräume (6) eine oder mehrere Versorgungsöffnungen (61) besitzt durch die die Verdampfermembranen (2) und/oder Kapillare (3) mit der zu verdampfenden Substanz in Verbindung stehen.

6. Verfahren nach Anspruch 5, wobei der Hohlraum (6) mindestens eine weitere Öffnung zum Druckausgleich (62) aufweist, die in Kontakt zum Strömungskanal (11) innerhalb des Hohlkörpers (1) und/oder zum Atmosphärendruck außerhalb des Hohlkörpers (1) steht und vorzugsweise nicht von der zu verdampfenden Substanz passierbar ist, wobei die zu verdampfende Substanz den Hohlraum (6) mindestens teilweise, vorzugsweise aber vollständig ausfüllt.

7. Verfahren nach einem der Ansprüche 5 oder 6, wobei sich zwischen dem oder den Hohlräumen (6) und dem Strömungskanal (11) und/oder den Kapillaren (3) und/oder Verdampfermembranen (2) eine oder mehrere entfernbare Verschlüsse (611, 621) befinden.

8. Verfahren nach einem der Ansprüche 5, 6 oder 7, wobei der oder die Hohlräume (6) durch eine entfernbare Trennwand (63) in eine Substanzversorgungskammer (631) und eine Substanzspeicherkammer (632) unterteilt sind.

9. Verfahren nach einem der Ansprüche 5, 6, 7 oder 8, wobei der oder die Hohlräume (6) mindestens ein Speichermedium (64) zum Speichern der zu verdampfenden Substanz enthalten, wobei das Speichermedium (die Speichermedien) (64) bevorzugt ein Schaum oder Vlies ist (sind).

10. Vorrichtung zur Ausführung des Verfahrens nach einem der Ansprüche 1 bis 9, umfassend einen vorzugsweise zylindrischen Hohlkörper (1) in welchem ein Strömungskanal (11) ausgebildet ist, mindestens eine im Strömungskanal (11) angeordnete Verdampfermembran (2), wobei die mindestens eine Verdampfermembran (2) mit einer mittels Um- und/oder Durchströmen eines Fluides verdampfbaren Wirk- und oder Aromastoff enthaltenden Substanz benetzt ist und/oder benetzbar ist.

11. Vorrichtung nach Anspruch 10, weiterhin umfassend mindestens eine im Strömungskanal (11) angeordnete Filtermembran (4, 5) zum Herausfiltern unerwünschter Bestandteile aus dem Fluidstrom, wobei die Filtermembran (4, 5) ggf. einen Katalysator beinhaltet um unerwünschte Bestandteile in unbedenkliche Produkte aufzuspalten.

12. Vorrichtung nach Anspruch 10 oder 11, wobei die Membran(en) (2, 4, 5) aus ein- oder mehrschichtigen Membranen, permeablen oder semipermeablen Membranen oder Kombination davon ausgewählt ist(sind) und orthogonal, winklig und/oder parallel zum Fluidstrom angeordnet ist(sind).

13. Vorrichtung nach Anspruch 10, 11 oder 12, weiterhin umfassend einen oder mehrere, mindestens teilweise, vorzugsweise aber vollständig von der verdampfbaren Substanz ausgefüllte(n) Hohlraum(Hohlräume) (6), wobei die verdampfbare Substanz durch eine oder mehrere Versorgungsöffnungen (61) mit der(den) Verdampfermembran(en) (2) direkt oder und/oder über Kapillare (3) in Kontakt steht.

14. Vorrichtung nach Anspruch 12, wobei der oder die Hohlräume (6) mindestens eine weitere Öffnung zum Druckausgleich (62) aufweist, die in Kontakt zum Strömungskanal (11) innerhalb des Hohlkörpers (1) und/oder zum Atmosphärendruck außerhalb des Hohlkörpers (1) steht und vorzugsweise so ausgestaltet ist, dass sie nicht von der verdampfbaren Substanz passierbar ist.

15. Vorrichtung nach einem der Ansprüche 10 bis 14, wobei der Hohlkörper (1) und falls vorhanden der Hohlraum (die Hohlräume) (6) aus einer oder mehreren ein- und/oder mehrlagigen Folien (12, 65) gebildet sind.

16. Vorrichtung nach einem der Ansprüche 12 bis 14, wobei sich zwischen dem oder den Hohlräumen (6) und dem Strömungskanal (11) und/oder den Kapillaren (3) und/oder Verdampfermembranen (2) eine oder mehrere entfernbare Verschlüsse (611, 621) befinden oder der Hohlraum (die Hohlräume) (6) durch eine entfernbare Trennwand (63) in eine Substanzversorgungskammer (631) und eine Substanzspeicherkammer (632) unterteilt sind.
